# EUROPÄISCHE PATENTANMELDUNG

(11) **EP 1 477 485 A1**
(43) Veröffentlichungstag der Anmeldung: **17.11.2004**
(21) Anmeldenummer: 04012513.0
(22) Anmeldetag: 02.10.1997
(51) Int. Cl.: C07D 333/70, C07D 333/24, C07D 333/40, C07D 307/68, C07D 307/24, C07D 277/56, C07D 213/80, C07D 213/79, C07D 231/14, C07D 231/16, C07D 327/06, A01N 43/18

(54) **Aminophenolderivate**

(30) Priorität: 15.10.1996 DE 19642529
(62) Teilanmeldung aus: 97912100.1
(71) Anmelder: Bayer CropScience AG, 40789 Monheim (DE)
(72) Erfinder: Krüger, Bernd-Wieland, Dr., 51467 Bergisch Gladbach (DE); Dehne, Heinz-Wilhelm, Prof. Dr. Dipl.-Ing. agr., 53125 Bonn (DE); Stenzel, Klaus, Dr., 40595 Düsseldorf (DE)

(57) **Zusammenfassung**

Die Erfindung betrifft neue Aminophenolderivate, ein Verfahren zu ihrer Herstellung und ihre Verwendung als Fungizide.

## Beschreibung

Die Erfindung betrifft neue Aminophenolderivate, ein Verfahren zu ihrer Herstellung und ihre Verwendung als Fungizide.

Es ist bereits bekannt geworden, daß bestimmte Aminophenolderivate eine fungizide Wirkung aufweisen (vergleiche z. B. EP-A 293718).

Die Wirkung dieser vorbekannten Verbindungen ist jedoch insbesondere bei niedrigen Aufwandmengen und Konzentrationen nicht in allen Anwendungsgebieten völlig zufriedenstellend.

Es wurden nun die neuen Aminophenolderivate der allgemeinen Formel (I) gefunden 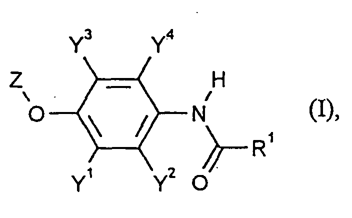 in welcher
- Y¹, Y², Y³ und Y⁴: unabhängig voneinander jeweils für Wasserstoff, Halogen, Alkyl, Halogenalkyl, Alkoxy, Halogenalkoxy, Alkylthio oder Halogenalkylthio stehen,
- R¹: für jeweils gegebenenfalls substituiertes Alkyl, Alkenyl, Aryl, Cycloalkyl, Cycloalkenyl oder Heterocyclyl steht und
- Z: für eine Gruppierung 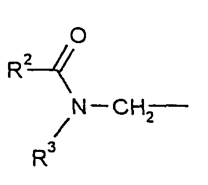 steht,
in welcher
R² für jeweils gegebenenfalls durch Sauerstoffatome unterbrochenes Alkyl, Alkoxy oder Alkylthio steht und
R³ für gegebenenfalls durch Sauerstoffatome unterbrochenes Alkyl steht, oder
- Z: für eine Gruppierung R⁴-O-CH₂- steht,
in welcher
R⁴ für gegebenenfalls durch Sauerstoffatome unterbrochenes Alkyl steht, oder
- Z: für eine Gruppierung 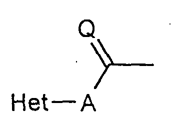 steht,
in welcher
Het für gegebenenfalls substituiertes Heterocyclyl steht,
A für eine Einfachbindung, Sauerstoff, Schwefel, Alkandiyl, oder für 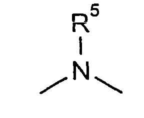 steht,
wobei
R⁵ für Wasserstoff oder Alkyl steht, und
Q für Sauerstoff oder Schwefel steht.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt. Sind die Kohlenwasserstoffketten durch Sauerstoffatome unterbrochen, so stehen die Sauerstoffatome nicht endständig und zwischen zwei Sauerstoffatomen in einer Kette stehen immer mindestens zwei Kohlenstoffatome.

Halogen steht im allgemeinen für Fluor, Chlor, Brom oder Iod, vorzugsweise für Fluor, Chlor oder Brom, insbesondere für Fluor oder Chlor.

Aryl steht für aromatische, mono- oder polycyclische Kohlenwasserstoffringe, wie z.B. Phenyl, Naphthyl, Anthranyl, Phenanthryl, vorzugsweise Phenyl oder Naphthyl, insbesondere Phenyl.

Heterocyclyl steht für gesättigte oder ungesättigte, sowie aromatische, ringförmige Verbindungen, in denen mindestens ein Ringglied ein Heteroatom, d. h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel. Enthält der Ring mehrere Sauerstoffatome, stehen diese nicht benachbart. Gegebenenfalls bilden die ringförmigen Verbindungen mit weiteren carbocyclischen oder heterocyclischen, ankondensierten oder überbrückten Ringen gemeinsam ein polycyclisches Ringsystem. Bevorzugt sind mono- oder bicyclische Ringsysteme, insbesondere mono- oder bicyclische, aromatische Ringsysteme.

Cycloalkyl steht für gesättigte, carbocyclische, ringförmige Verbindungen, die gegebenenfalls mit weiteren carbocyclischen, ankondensierten oder überbrückten Ringen ein polycyclisches Ringsystem bilden.

Cycloalkenyl steht für carbocyclische, ringförmige Verbindungen, die mindestens eine Doppelbindung enthalten und gegebenenfalls mit weiteren carbocyclischen, ankondensierten oder überbrückten Ringen ein polycyclisches Ringsystem bilden.

Weiterhin wurde gefunden, daß man die neuen Verbindungen der allgemeinen Formel (I) erhält, wenn man a) Hydroxyverbindungen der allgemeinen Formel (II) 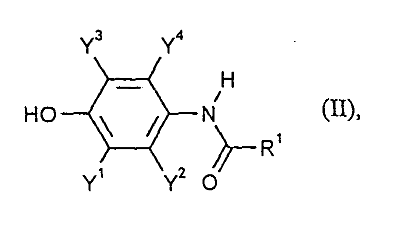 in welcher
- R¹, Y¹, Y², Y³ und Y⁴: die oben angegebenen Bedeutungen haben,
mit einer Halogenverbindung der allgemeinen Formel (III)

Z-X¹ (III),

in welcher
- Z: die oben angegebene Bedeutung hat und
- X¹: für Halogen steht,
gegebenenfalls in Gegenwart eines Säureakzeptors und gegebenenfalls in Gegenwart eines Verdünnungsmittels, umsetzt oder wenn man b)

Aminoverbindungen der allgemeinen Formel (IV) 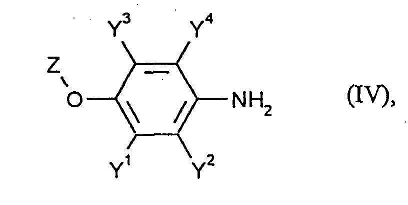 in welcher
- Y¹, Y², Y³, Y⁴ und Z: die oben angegebenen Bedeutungen haben,
mit einem Carbonsäurederivat der allgemeinen Formel (V) 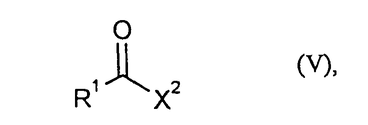 in welcher
- R¹: die oben angegebene Bedeutung hat und
- X²: für Halogen oder 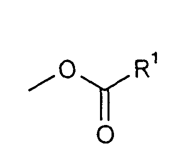 steht,
gegebenenfalls in Gegenwart eines Säureakzeptors und gegebenenfalls in Gegenwart eines Verdünnungsmittels, umsetzt.

Schließlich wurde gefunden, daß die neuen Verbindungen der allgemeinen Formel (I) eine sehr starke fungizide Wirkung zeigen.

Die erfindungsgemäßen Verbindungen liegen gegebenenfalls als Mischungen verschiedener möglicher isomerer Formen, insbesondere von Stereoisomeren, wie z.B. E- und Z-, threo- und erythro-, sowie optischen Isomeren vor. Es werden sowohl die E- als auch die Z-Isomeren, wie auch die threo- und erythro-, sowie die optischen Isomeren sowie beliebige Mischungen dieser Isomeren beansprucht.

Gegenstand der Erfindung sind vorzugsweise Verbindungen der Formel (I),
in welcher
- Y¹: für Wasserstoff, Halogen, Alkyl, Alkoxy, Alkylthio mit jeweils 1 bis 4 Kohlenstoffatomen, Halogenalkyl, Halogenalkoxy, Halogenalkylthio mit jeweils 1 bis 3 Kohlenstoffatomen und 1 bis 5 gleichen oder verschiedenen Halogenatomen,
- Y²: für Wasserstoff, Halogen, Alkyl, Alkoxy, Alkylthio mit jeweils 1 bis 4 Kohlenstoffatomen, Halogenalkyl, Halogenalkoxy, Halogenalkylthio mit jeweils 1 bis 3 Kohlenstoffatomen und 1 bis 5 gleichen oder verschiedenen Halogenatomen,
- Y³: für Wasserstoff, Halogen, Alkyl, Alkoxy, Alkylthio mit jeweils 1 bis 4 Kohlenstoffatomen, Halogenalkyl, Halogenalkoxy, Halogenalkylthio mit jeweils 1 bis 3 Kohlenstoffatomen und 1 bis 5 gleichen oder verschiedenen Halogenatomen,
- Y⁴: für Wasserstoff, Halogen, Alkyl, Alkoxy, Alkylthio mit jeweils 1 bis 4 Kohlenstoffatomen, Halogenalkyl, Halogenalkoxy, Halogenalkylthio mit jeweils 1 bis 3 Kohlenstoffatomen und 1 bis 5 gleichen oder verschiedenen Halogenatomen,
wobei mindestens zwei der Reste Y¹ bis Y⁴ für Halogen stehen,
- R¹: für jeweils gegebenenfalls einfach bis fünffach substituiertes Alkyl oder Alkenyl mit jeweils 4 bis 10 Kohlenstoffatomen steht, und die möglichen Substituenten vorzugsweise aus der nachstehenden Aufzählung ausgewählt sind: Halogen, Alkoxy mit 1 bis 4 Kohlenstoffatomen, Alkylthio mit 1 bis 4 Kohlenstoffatomen, Halogenalkoxy mit 1 bis 2 Kohlenstoffatomen und 1 bis 5 Halogenatomen, Halogenalkylthio mit 1 bis 2 Kohlenstoffatomen und 1 bis 5 Halogenatomen oder gegebenenfalls durch Halogen oder Alkyl substituiertes Cycloalkyl mit 3 bis 6 Kohlenstoffatomen, wobei das Kohlenstoffatom, das an die Carbonylgruppe gebunden ist, kein Wasserstoffatom trägt, oder
- R¹: für jeweils gegebenenfalls einfach bis fünffach substituiertes Cycloalkyl oder Cycloalkenyl mit jeweils 3 bis 12 Kohlenstoffatomen steht, und die möglichen Substituenten vorzugsweise aus der nachstehenden Aufzählung ausgewählt sind: Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 2 Kohlenstoffatomen und 1 bis 5 Halogenatomen, Halogenalkoxy mit 1 bis 2 Kohlenstoffatomen und 1 bis 5 Halogenatomen, Halogenalkylthio mit 1 bis 2 Kohlenstoffatomen und 1 bis 5 Halogenatomen oder gegebenenfalls durch Halogen oder Alkyl substituiertes Cycloalkyl mit 3 bis 6 Kohlenstoffatomen, und
- Z: für eine Gruppierung 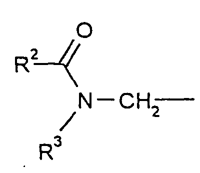 steht,
in welcher
R² für jeweils gegebenenfalls durch 1 bis 2 Sauerstoffatome unterbrochenes Alkyl, Alkoxy oder Alkylthio mit 1 bis 8 Kohlenstoffatomen steht und
R³ für gegebenenfalls durch 1 bis 2 Sauerstoffatome unterbrochenes Alkyl mit 1 bis 8 Kohlenstoffatomen steht, oder
- Z: für eine Gruppierung R⁴-O-CH₂- steht,
in welcher
R⁴ für gegebenenfalls durch 1 bis 2 Sauerstoffatome unterbrochenes Alkyl mit 1 bis 8 Kohlenstoffatomen steht.

Ebenfalls vorzugsweise Gegenstand der vorliegenden Erfindung sind Verbindungen der Formel (I),
in welcher
- Y¹: für Wasserstoff, Halogen, Alkyl, Alkoxy, Alkylthio mit jeweils 1 bis 4 Kohlenstoffatomen, Halogenalkyl, Halogenalkoxy, Halogenalkylthio mit jeweils 1 bis 3 Kohlenstoffatomen und 1 bis 5 gleichen oder verschiedenen Halogenatomen,
- Y²: für Wasserstoff, Halogen, Alkyl, Alkoxy, Alkylthio mit jeweils 1 bis 4 Kohlenstoffatomen, Halogenalkyl, Halogenalkoxy, Halogenalkylthio mit jeweils 1 bis 3 Kohlenstoffatomen und 1 bis 5 gleichen oder verschiedenen Halogenatomen,
- Y³: für Wasserstoff, Halogen, Alkyl, Alkoxy, Alkylthio mit jeweils 1 bis 4 Kohlenstoffatomen, Halogenalkyl, Halogenalkoxy, Halogenalkylthio mit jeweils 1 bis 3 Kohlenstoffatomen und 1 bis 5 gleichen oder verschiedenen Halogenatomen,
- Y⁴: für Wasserstoff, Halogen, Alkyl, Alkoxy, Alkylthio mit jeweils 1 bis 4 Kohlenstoffatomen, Halogenalkyl, Halogenalkoxy, Halogenalkylthio mit jeweils 1 bis 3 Kohlenstoffatomen und 1 bis 5 gleichen oder verschiedenen Halogenatomen,
wobei mindestens zwei der Reste Y¹ bis Y⁴ für Halogen stehen,
- R¹: für jeweils gegebenenfalls einfach bis fünffach substituiertes Alkyl oder Alkenyl mit jeweils 4 bis 10 Kohlenstoffatomen steht, und die möglichen Substituenten vorzugsweise aus der nachstehenden Aufzählung ausgewählt sind: Halogen, Alkoxy mit 1 bis 4 Kohlenstoffatomen, Alkylthio mit 1 bis 4 Kohlenstoffatomen, Halogenalkoxy mit 1 bis 2 Kohlenstoffatomen und 1 bis 5 Halogenatomen, Halogenalkylthio mit 1 bis 2 Kohlenstoffatomen und 1 bis 5 Halogenatomen oder gegebenenfalls durch Halogen oder Alkyl substituiertes Cycloalkyl mit 3 bis 6 Kohlenstoffatomen, wobei das Kohlenstoffatom, das an die Carbonylgruppe gebunden ist, kein Wasserstoffatom trägt, oder
- R¹: für jeweils gegebenenfalls einfach bis fünffach substituiertes Cycloalkyl oder Cycloalkenyl mit jeweils 3 bis 12 Kohlenstoffatomen steht, und die' möglichen Substituenten vorzugsweise aus der nachstehenden Aufzählung ausgewählt sind: Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 2 Kohlenstoffatomen und 1 bis 5 Halogenatomen, Halogenalkoxy mit 1 bis 2 Kohlenstoffatomen und 1 bis 5 Halogenatomen, Halogenalkylthio mit 1 bis 2 Kohlenstoffatomen und 1 bis 5 Halogenatomen oder gegebenenfalls durch Halogen oder Alkyl substituiertes Cycloalkyl mit 3 bis 6 Kohlenstoffatomen, und
- Z: für eine Gruppierung 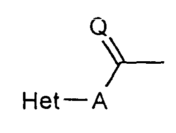 steht,
in welcher
Het für gegebenenfalls einfach bis dreifach durch Halogen, Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen substituiertes Heterocyclyl mit 3 bis 7 Ringgliedern, von denen jeweils 1 bis 3 gleiche oder verschiedene Heteroatome - insbesondere Stickstoff, Sauerstoff und/oder Schwefel - sind, steht,
A für eine Einfachbindung, Sauerstoff, Schwefel, Alkandiyl mit 1 bis 2 Kohlenstoffatomen, oder für 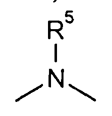 steht,
wobei
R⁵ für Wasserstoff oder Alkyl mit 1 bis 4 Kohlenstoffatomen steht, und
Q für Sauerstoff oder Schwefel steht.

Die Erfindung betrifft insbesondere Verbindungen der Formel (I),
in welcher
- Y¹: für Wasserstoff, Fluor, Chlor, Brom, Methyl, Methoxy, Ethoxy, Methylthio, Ethylthio, Trifluormethyl, Difluormethyl, Fluormethyl, Fluorchlormethyl, Difluormethoxy, Trifluormethoxy, Difluorchlormethoxy, Difluormethylthio, Difluorchlormethylthio, Trifluormethylthio steht,
- Y²: für Wasserstoff, Fluor, Chlor, Brom, Methyl, Methoxy, Ethoxy, Methylthio, Ethylthio, Trifluormethyl, Difluormethyl, Fluormethyl, Fluorchlormethyl, Difluormethoxy, Trifluormethoxy, Difluorchlormethoxy, Difluormethylthio, Difluorchlormethylthio, Trifluormethylthio steht,
- Y³: für Wasserstoff, Fluor, Chlor, Brom, Methyl, Methoxy, Ethoxy, Methylthio, Ethylthio, Trifluormethyl, Difluormethyl, Fluormethyl, Fluorchlormethyl, Difluormethoxy, Trifluormethoxy, Difluorchlormethoxy, Difluormethylthio, Difluorchlormethylthio, Trifluormethylthio steht,
- Y⁴: für Wasserstoff, Fluor, Chlor, Brom, Methyl, Methoxy, Ethoxy, Methylthio, Ethylthio, Trifluormethyl, Difluormethyl, Fluormethyl, Fluorchlormethyl, Difluormethoxy, Trifluormethoxy, Difluorchlormethoxy, Difluormethylthio, Difluorchlormethylthio, Trifluormethylthio steht,
wobei mindestens zwei der Reste Y¹ bis Y⁴ jeweils für Fluor, Chlor oder Brom stehen,
- R¹: für 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl steht, wobei die genannten Alkylreste gegebenenfalls durch 1 bis 3 Halogenatome, vorzugsweise Fluor, Chlor oder Brom, substituiert sind, oder
- R¹: für 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-1-methyl-2-propenyl, 1-Ethyl-2-methyl-1-propenyl und 1-Ethyl-2-methyl-2-propenyl steht, wobei die genannten Alkylreste gegebenenfalls durch 1 bis 3 Halogenatome, vorzugsweise Fluor, Chlor oder Brom, substituiert sind, oder
- R¹: für Cyclopropyl, Cyclobutyl, Cyclopentyl, Cyclohexyl, Cycloheptyl, 1-Cyclopentenyl, 2-Cyclopentenyl, 3-Cyclopentenyl, 1,3-Cyclopentadienyl, 1,4-Cyclopentadienyl, 1-Cyclohexenyl, 2-Cyclohexenyl, 3-Cyclohexenyl, 1,3-Cyclohexadienyl, 1,4-Cyclohexadienyl, 1,5-Cyclohexadienyl, 2,4-Cyclohexadienyl, 1-Cycloheptenyl, 2-Cycloheptenyl, 3-Cycloheptenyl, 4-Cycloheptenyl, 1,3-Cycloheptadienyl, 1,4-Cycloheptadienyl, 1,5-Cycloheptadienyl, 1,6-Cycloheptadienyl, 2,4-Cycloheptadienyl, 2,5-Cycloheptadienyl, 2,6-Cycloheptadienyl und 3,5-Cycloheptadienyl steht, wobei die genannten Reste gegebenenfalls durch 1 bis 3 Halogenatome, vorzugsweise Chlor, Fluor oder Brom, und/oder Methyl oder Ethyl substituiert sind, oder
- R¹: für Bicyclo-[2.2.1]-hex-5-yl, Bicyclo-[2.2.1]-hept-2-yl, Bicycylo-[2.2.2]-oct-2-yl, Bicyclo-[3.2.1]-oct-6-yl, Bicyclo-[3.2.2]-non-6-yl, Bicyclo-[4.2.2]-dec-7-yl, Bicyclo-[3.1.0]-hex-1-yl, Bicyclo-[4.1.0]-hept-1-yl, Bicyclo-[4.3.0]-non-1-yl, Bicyclo-[4.4.0]-dec-1-yl, besonders bevorzugt für 5-Methyl-bicyclo-[2.1.1]-hex-5-yl, 2-Methyl-bicyclo-[2.2.1]-hept-2-yl, 2-Methyl-bicyclo-[2.2.2]-oct-2-yl, 6-Methyl-bicyclo-[3.2.1]-oct-6-yl, 6-Methyl-bicyclo-[3.2.2]-non-6-yl, 7-Methyl-bicyclo-[4.2.2]-dec-7-yl, 1-Methyl-bicyclo-[3.1.0]-hex-1-yl, 1-Methyl-bicyclo-[4.1.0]-hept-1-yl, 1-Methyl-bicyclo-[4.3.0]-non-1-yl, 1-Methyl-bicyclo-[4.4.0]-dec-1-yl, 2-Methyl-bicyclo-[3.1.0]-hex-1-yl, 2-Methyl-bicyclo-[4.1.0]-hept-1-yl, 2-Methyl-bicyclo-[4.3.0]-non-1-yl, 2-Methyl-bicyclo-[4.4.0]-dec-1-yl, Bicyclo-[2.2.1]-hept-2-en-5-yl, Bicyclo-[2.2.2]-oct-2-en-5-yl, Bicyclo-[4.2.2]-dec-7-en-2-yl, Bicyclo-[4.3.0]-non-7-en-1-yl, Bicyclo-[4.4.0]-dec-3-en-1-yl, Bicyclo-[4.1.0]-hept-3-en-1-yl, 5-Methyl-bicyclo-[2.2.1]-hept-2-en-5-yl, 5-Methyl-bicylo-[2.2.2]-oct-2-en-5-yl, 2-Methyl-bicyclo-[4.2.2]-dec-7-en-2-yl, 2-Methyl-bicyclo-[4.3.0]-non-7-en-1-yl, 2-Methyl-bicyclo-[4.4.0]-dec-3-en-1-yl und 2-Methyl-bicyclo-[4.1.0]-hept-3-en-1-yl steht,
- Z: für eine Gruppierung 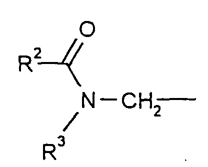 steht,
in welcher
R² für jeweils gegebenenfalls durch ein Sauerstoffatom unterbrochenes Alkyl oder Alkoxy mit 1 bis 6 Kohlenstoffatomen steht und
R³ für gegebenenfalls durch ein Sauerstoffatom unterbrochenes Alkyl mit 1 bis 6 Kohlenstoffatomen steht, oder
- Z: für eine Gruppierung R⁴-O-CH₂- steht,
in welcher
R⁴ für gegebenenfalls durch ein Sauerstoffatom unterbrochenes Alkyl mit 1 bis 6 Kohlenstoffatomen steht.

Steht R¹ für gegebenenfalls substituiertes Alkyl, ist es insbesondere so substituiert, daß das Kohlenstoffatom, das an die Carbonylgruppe gebunden ist, kein Wasserstoffatom mehr trägt.

Die Erfindung betrifft auch insbesondere Verbindungen der Formel (I),
in welcher
- Y¹: für Wasserstoff, Fluor, Chlor, Brom, Methyl, Methoxy, Ethoxy, Methylthio, Ethylthio, Trifluormethyl, Difluormethyl, Fluormethyl, Fluorchlormethyl, Difluormethoxy, Trifluormethoxy, Difluorchlormethoxy, Difluormethylthio, Difluorchlormethylthio, Trifluormethylthio steht,
- Y²: für Wasserstoff, Fluor, Chlor, Brom, Methyl, Methoxy, Ethoxy, Methylthio, Ethylthio, Trifluormethyl, Difluormethyl, Fluormethyl, Fluorchlormethyl, Difluormethoxy, Trifluormethoxy, Difluorchlormethoxy, Difluormethylthio, Difluorchlormethylthio, Trifluormethylthio steht,
- Y³: für Wasserstoff, Fluor, Chlor, Brom, Methyl, Methoxy, Ethoxy, Methylthio, Ethylthio, Trifluormethyl, Difluormethyl, Fluormethyl, Fluorchlormethyl, Difluormethoxy, Trifluormethoxy, Difluorchlormethoxy, Difluormethylthio, Difluorchlormethylthio, Trifluormethylthio steht,
- Y⁴: für Wasserstoff, Fluor, Chlor, Brom, Methyl, Methoxy, Ethoxy, Methylthio, Ethylthio, Trifluormethyl, Difluormethyl, Fluormethyl, Fluorchlormethyl, Difluormethoxy, Trifluormethoxy, Difluorchlormethoxy, Difluormethylthio, Difluorchlormethylthio, Trifluormethylthio steht,
wobei mindestens zwei der Reste Y¹ bis Y⁴ jeweils für Fluor, Chlor oder Brom stehen,
- R¹: für 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl steht, wobei die genannten Alkylreste gegebenenfalls durch 1 bis 3 Halogenatome, vorzugsweise Fluor, Chlor oder Brom, substituiert sind, oder
- R¹: für 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-1-methyl-2-propenyl, 1-Ethyl-2-methyl-1-propenyl und 1-Ethyl-2-methyl-2-propenyl steht, wobei die genannten Alkylreste gegebenenfalls durch 1 bis 3 Halogenatome, vorzugsweise Fluor, Chlor oder Brom, substituiert sind, oder
- R¹: für Cyclopropyl, Cyclobutyl, Cyclopentyl, Cyclohexyl, Cycloheptyl, 1-Cyclopentenyl, 2-Cyclopentenyl, 3-Cyclopentenyl, 1,3-Cyclopentadienyl, 1,4-Cyclopentadienyl, 1-Cyclohexenyl, 2-Cyclohexenyl, 3-Cyclohexenyl, 1,3-Cyclohexadienyl, 1,4-Cyclohexadienyl, 1,5-Cyclohexadienyl, 2,4-Cyclohexadienyl, 1-Cycloheptenyl, 2-Cycloheptenyl, 3-Cycloheptenyl, 4-Cycloheptenyl, 1,3-Cycloheptadienyl, 1,4-Cycloheptadienyl, 1,5-Cycloheptadienyl, 1,6-Cycloheptadienyl, 2,4-Cycloheptadienyl, 2,5-Cycloheptadienyl, 2,6-Cycloheptadienyl und 3,5-Cycloheptadienyl steht, wobei die genannten Reste gegebenenfalls durch 1 bis 3 Halogenatome, vorzugsweise Chlor, Fluor oder Brom, und/oder Methyl oder Ethyl substituiert sind, oder
- R¹: für Bicyclo-[2.2.1]-hex-5-yl, Bicyclo-[2.2.1]-hept-2-yl, Bicycylo-[2.2.2]-oct-2-yl, Bicyclo-[3.2.1]-oct-6-yl, Bicyclo-[3.2.2]-non-6-yl, Bicyclo-[4.2.2]-dec-7-yl, Bicyclo-[3.1.0]-hex-1-yl, Bicyclo-[4.1.0]-hept-1-yl, Bicyclo-[4.3.0]-non-1-yl, Bicyclo-[4.4.0]-dec-1-yl, besonders bevorzugt für 5-Methyl-bicyclo-[2.1.1]-hex-5-yl, 2-Methyl-bicyclo-[2.2.1]-hept-2-yl, 2-Methyl-bicyclo-[2.2.2]-oct-2-yl, 6-Methyl-bicyclo-[3.2.1]-oct-6-yl, 6-Methyl-bicyclo-[3.2.2]-non-6-yl, 7-Methyl-bicyclo-[4.2.2]-dec-7-yl, 1-Methyl-bicyclo-[3.1.0]-hex-1-yl, 1-Methyl-bicyclo-[4.1.0]-hept-1-yl, 1-Methyl-bicyclo-[4.3.0]-non-1-yl, 1-Methyl-bicyclo-[4.4.0]-dec-1-yl, 2-Methyl-bicyclo-[3.1.0]-hex-1-yl, 2-Methyl-bicyclo-[4.1.0]-hept-1-yl, 2-Methyl-bicyclo-[4.3.0]-non-1-yl, 2-Methyl-bicyclo-[4.4.0]-dec-1-yl, Bicyclo-[2.2.1]-hept-2-en-5-yl, Bicyclo-[2.2.2]-oct-2-en-5-yl, Bicyclo-[4.2.2]-dec-7-en-2-yl, Bicyclo-[4.3.0]-non-7-en-1-yl, Bicyclo-[4.4.0]-dec-3-en-1-yl, Bicyclo-[4.1.0]-hept-3-en-1-yl, 5-Methyl-bicyclo-[2.2.1]-hept-2-en-5-yl, 5-Methyl-bicyclo-[2.2.2]-oct-2-en-5-yl, 2-Methyl-bicyclo-[4.2.2]-dec-7-en-2-yl, 2-Methyl-bicyclo-[4.3.0]-non-7-en-1-yl, 2-Methyl-bicyclo-[4.4.0]-dec-3-en-1-yl und 2-Methyl-bicyclo-[4.1.0]-hept-3-en-1-yl steht,
- Z: für eine Gruppierung 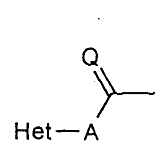 steht,
in welcher
Het für gegebenenfalls einfach bis dreifach durch Halogen, Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen substituiertes Heterocyclyl mit 3 bis 7 Ringgliedern, von denen jeweils 1 bis 3 gleiche oder verschiedene Heteroatome - insbesondere Stickstoff, Sauerstoff und/oder Schwefel - sind, steht,
A für eine Einfachbindung, Sauerstoff, Schwefel, Methylen, oder für 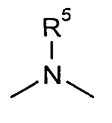 steht,
wobei
R⁵ für Wasserstoff, Methyl oder Ethyl steht, und
Q für Sauerstoff oder Schwefel steht.

Steht R¹ für gegebenenfalls substituiertes Alkyl, ist es insbesondere so substituiert, daß das Kohlenstoffatom, das an die Carbonylgruppe gebunden ist, kein Wasserstoffatom mehr trägt.

Ganz besonders bevorzugt sind Verbindungen der Formel (I),
in welcher
- Y¹: für Wasserstoff, Fluor, Chlor, Brom, Methyl, Methoxy, Ethoxy, Trifluormethyl, Difluormethyl, Fluormethyl, Fluorchlormethyl, Difluormethoxy, Trifluormethoxy, Difluorchlormethoxy steht,
- Y²: für Wasserstoff, Fluor, Chlor, Brom, Methyl, Methoxy, Ethoxy, Trifluormethyl, Difluormethyl, Fluormethyl, Fluorchlormethyl, Difluormethoxy, Trifluormethoxy, Difluorchlormethoxy steht,
- Y³: für Wasserstoff, Fluor, Chlor, Brom, Methyl, Methoxy, Ethoxy, Trifluormethyl, Difluormethyl, Fluormethyl, Fluorchlormethyl, Difluormethoxy, Trifluormethoxy, Difluorchlormethoxy steht,
- Y⁴: für Wasserstoff, Fluor, Chlor, Brom, Methyl, Methoxy, Ethoxy, Trifluormethyl, Difluormethyl, Fluormethyl, Fluorchlormethyl, Difluormethoxy, Trifluormethoxy, Difluorchlormethoxy steht,
wobei mindestens zwei der Reste Y¹ bis Y⁴ jeweils für Fluor, Chlor oder Brom stehen,
- R¹: für 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl steht, wobei die genannten Alkylreste gegebenenfalls durch 1 bis 3 Halogenatome, vorzugsweise Fluor, Chlor oder Brom, substituiert sind, oder
- R¹: für 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-1-methyl-2-propenyl, 1-Ethyl-2-methyl-1-propenyl und 1-Ethyl-2-methyl-2-propenyl steht, wobei die genannten Alkylreste gegebenenfalls durch 1 bis 3 Halogenatome, vorzugsweise Fluor, Chlor oder Brom, substituiert sind, oder
- R¹: für Cyclopropyl, Cyclobutyl, Cyclopentyl, Cyclohexyl, Cycloheptyl, 1-Cyclopentenyl, 2-Cyclopentenyl, 3-Cyclopentenyl, 1,3-Cyclopentadienyl, 1,4-Cyclopentadienyl, 1-Cyclohexenyl, 2-Cyclohexenyl, 3-Cyclohexenyl, 1,3-Cyclohexadienyl, 1,4-Cyclohexadienyl, 1,5-Cyclohexadienyl, 2,4-Cyclohexadienyl, 1-Cycloheptenyl, 2-Cycloheptenyl, 3-Cycloheptenyl, 4-Cycloheptenyl, 1,3-Cycloheptadienyl, 1,4-Cycloheptadienyl, 1,5-Cycloheptadienyl, 1,6-Cycloheptadienyl, 2,4-Cycloheptadienyl, 2,5-Cycloheptadienyl, 2,6-Cycloheptadienyl und 3,5-Cycloheptadienyl steht, wobei die genannten Reste gegebenenfalls durch 1 bis 3 Halogenatome, vorzugsweise Chlor, Fluor oder Brom, und/oder Methyl oder Ethyl substituiert sind, oder
- R¹: für Bicyclo-[2.2.1]-hex-5-yl, Bicyclo-[2.2.1]-hept-2-yl, Bicycylo-[2.2.2]-oct-2-yl, Bicyclo-[3.2.1]-oct-6-yl, Bicyclo-[3.2.2]-non-6-yl, Bicyclo-[4.2.2]-dec-7-yl, Bicyclo-[3.1.0]-hex-1-yl, Bicyclo-[4.1.0]-hept-1-yl, Bicyclo-[4.3.0]-non-1-yl, Bicyclo-[4.4.0]-dec-1-yl, besonders bevorzugt für 5-Methyl-bicyclo-[2.1.1]-hex-5-yl, 2-Methyl-bicyclo-[2.2.1]-hept-2-yl, 2-Methyl-bicyclo-[2.2.2]-oct-2-yl, 6-Methyl-bicyclo-[3.2.1]-oct-6-yl, 6-Methyl-bicyclo-[3.2.2]-non-6-yl, 7-Methyl-bicyclo-[4.2.2]-dec-7-yl, 1-Methyl-bicyclo-[3.1.0]-hex-1-yl, 1-Methyl-bicyclo-[4.1.0]-hept-1-yl, 1-Methyl-bicyclo-[4.3.0]-non-1-yl, 1-Methyl-bicyclo-[4.4.0]-dec-1-yl, 2-Methyl-bicyclo-[3.1.0]-hex-1-yl, 2-Methyl-bicyclo-[4.1.0]-hept-1-yl, 2-Methyl-bicyclo-[4.3.0]-non-1-yl, 2-Methyl-bicyclo-[4.4.0]-dec-1-yl, Bicyclo-[2.2.1]-hept-2-en-5-yl, Bicyclo-[2.2.2]-oct-2-en-5-yl, Bicyclo-[4.2.2]-dec-7-en-2-yl, Bicyclo-[4.3.0]-non-7-en-1-yl, Bicyclo-[4.4.0]-dec-3-en-1-yl, Bicyclo-[4.1.0]-hept-3-en-1-yl, 5-Methyl-bicyclo-[2.2.1]-hept-2-en-5-yl, 5-Methyl-bicyclo-[2.2.2]-oct-2-en-5-yl, 2-Methyl-bicyclo-[4.2.2]-dec-7-en-2-yl, 2-Methyl-bicyclo-[4.3.0]-non-7-en-1-yl, 2-Methyl-bicyclo-[4.4.0]-dec-3-en-1-yl und 2-Methyl-bicyclo-[4.1.0]-hept-3-en-1-yl steht, und
- Z: für eine Gruppierung 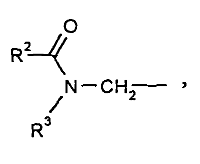 in welcher
R² für Methyl, Ethyl, n- oder i-Propyl, n-, i-, s- oder t-Butyl, Methoxyethyl, Methoxy, Ethoxy, n- oder i-Propoxy oder Methoxyethoxy steht und
R³ für Methyl, Ethyl, n- oder i-Propyl, n-, i-, s- oder t-Butyl oder Methoxyethyl steht oder
- Z: für eine Gruppierung R⁴-O-CH₂- steht,
in welcher
R⁴ für Methyl, Ethyl, n- oder i-Propyl, n-, i-, s- oder t-Butyl oder Methoxyethyl steht.

Steht R¹ für gegebenenfalls substituiertes Alkyl, ist es insbesondere so substituiert, daß das Kohlenstoffatom, das an die Carbonylgruppe gebunden ist, kein Wasserstoffatom mehr trägt.

Ganz besonders bevorzugt sind auch Verbindungen der Formel (I),
in welcher
- Y¹: für Wasserstoff, Fluor, Chlor, Brom, Methyl, Methoxy, Ethoxy, Trifluormethyl, Difluormethyl, Fluormethyl, Fluorchlormethyl, Difluormethoxy, Trifluormethoxy, Difluorchlormethoxy steht,
- Y²: für Wasserstoff, Fluor, Chlor, Brom, Methyl, Methoxy, Ethoxy, Trifluormethyl, Difluormethyl, Fluormethyl, Fluorchlormethyl, Difluormethoxy, Trifluormethoxy, Difluorchlormethoxy steht,
- Y³: für Wasserstoff, Fluor, Chlor, Brom, Methyl, Methoxy, Ethoxy, Trifluormethyl, Difluormethyl, Fluormethyl, Fluorchlormethyl, Difluormethoxy, Trifluormethoxy, Difluorchlormethoxy steht,
- Y⁴: für Wasserstoff, Fluor, Chlor, Brom, Methyl, Methoxy, Ethoxy, Trifluormethyl, Difluormethyl, Fluormethyl, Fluorchlormethyl, Difluormethoxy, Trifluormethoxy, Difluorchlormethoxy steht,
wobei mindestens zwei der Reste Y¹ bis Y⁴ jeweils für Fluor, Chlor oder' Brom stehen,
- R¹: für 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl steht, wobei die genannten Alkylreste gegebenenfalls durch 1 bis 3 Halogenatome, vorzugsweise Fluor, Chlor oder Brom, substituiert sind, oder
- R¹: für 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-1-methyl-2-propenyl, 1-Ethyl-2-methyl-1-propenyl und 1-Ethyl-2-methyl-2-propenyl steht, wobei die genannten Alkylreste gegebenenfalls durch 1 bis 3 Halogenatome, vorzugsweise Fluor, Chlor oder Brom, substituiert sind, oder
- R¹: für Cyclopropyl, Cyclobutyl, Cyclopentyl, Cyclohexyl, Cycloheptyl, 1-Cyclopentenyl, 2-Cyclopentenyl, 3-Cyclopentenyl, 1,3-Cyclopentadienyl, 1,4-Cyclopentadienyl, 1-Cyclohexenyl, 2-Cyclohexenyl, 3-Cyclohexenyl, 1,3-Cyclohexadienyl, 1,4-Cyclohexadienyl, 1,5-Cyclohexadienyl, 2,4-Cyclohexadienyl, 1-Cycloheptenyl, 2-Cycloheptenyl, 3-Cycloheptenyl, 4-Cycloheptenyl, 1,3-Cycloheptadienyl, 1,4-Cycloheptadienyl, 1,5-Cycloheptadienyl, 1,6-Cycloheptadienyl, 2,4-Cycloheptadienyl, 2,5-Cycloheptadienyl, 2,6-Cycloheptadienyl und 3,5-Cycloheptadienyl steht, wobei die genannten Reste gegebenenfalls durch 1 bis 3 Halogenatome, vorzugsweise Chlor, Fluor oder Brom, und/oder Methyl oder Ethyl substituiert sind, oder
- R¹: für Bicyclo-[2.2.1]-hex-5-yl, Bicyclo-[2.2.1]-hept-2-yl, Bicycylo-[2.2.2]-oct-2-yl, Bicyclo-[3.2.1]-oct-6-yl, Bicyclo-[3.2.2]-non-6-yl, Bicyclo-[4.2.2]-dec-7-yl, Bicyclo-[3.1.0]-hex-1-yl, Bicyclo-[4.1.0]-hept-1-yl, Bicyclo-[4.3.0]-non-1-yl, Bicyclo-[4.4.0]-dec-1-yl, besonders bevorzugt für 5-Methyl-bicyclo-[2.1.1]-hex-5-yl, 2-Methyl-bicyclo-[2.2.1]-hept-2-yl, 2-Methyl-bicyclo-[2.2.2]-oct-2-yl, 6-Methyl-bicyclo-[3.2.1]-oct-6-yl, 6-Methyl-bicyclo-[3.2.2]-non-6-yl, 7-Methyl-bicyclo-[4.2.2]-dec-7-yl, 1-Methyl-bicyclo-[3.1.0]-hex-1-yl, 1-Methyl-bicyclo-[4.1.0]-hept-1-yl, 1-Methyl-bicyclo-[4.3.0]-non-1-yl, 1-Methyl-bicyclo-[4.4.0]-dec-1-yl, 2-Methyl-bicyclo-[3.1.0]-hex-1-yl, 2-Methyl-bicyclo-[4.1.0]-hept-1-yl, 2-Methyl-bicyclo-[4.3.0]-non-1-yl, 2-Methyl-bicyclo-[4.4.0]-dec-1-yl, Bicyclo-[2.2.1]-hept-2-en-5-yl, Bicyclo-[2.2.2]-oct-2-en-5-yl, Bicyclo-[4.2.2]-dec-7-en-2-yl, Bicyclo-[4.3.0]-non-7-en-1-yl, Bicyclo-[4.4.0]-dec-3-en-1-yl, Bicyclo-[4.1.0]-hept-3-en-1-yl, 5-Methyl-bicyclo-[2.2.1]-hept-2-en-5-yl, 5-Methyl-bicyclo-[2.2.2]-oct-2-en-5-yl, 2-Methyl-bicyclo-[4.2.2]-dec-7-en-2-yl, 2-Methyl-bicyclo-[4.3.0]-non-7-en-1-yl, 2-Methyl-bicyclo-[4.4.0]-dec-3-en-1-yl und 2-Methyl-bicyclo-[4.1.0]-hept-3-en-1-yl steht, und
- Z: für eine Gruppierung 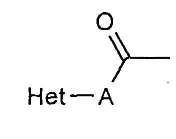 steht,
in welcher
Het für gegebenenfalls einfach bis dreifach durch Fluor, Chlor, Brom, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Trifluormethyl, Difluormethyl, Trifluormethoxy, Difluormethoxy, Difluorchlormethoxy, Trifluormethylthio, Difluormethylthio oder Difluorchlormethylthio, substituiertes Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, und
A für eine Einfachbindung, Sauerstoff, Schwefel, Methylen oder für 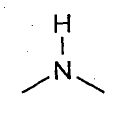 steht.

Steht R¹ für gegebenenfalls substituiertes Alkyl, ist es insbesondere so substituiert, daß das Kohlenstoffatom, das an die Carbonylgruppe gebunden ist, kein Wasserstoffatom mehr trägt.

Von ganz besonderm Interesse sind Verbindungen der Formel (I), in denen R¹ und Z die oben angegebenen Bedeutungen haben und in denen
- Y¹ und Y²: für Chlor stehen,
- Y³: für Wasserstoff oder Chlor steht und
- Y⁴: für Wasserstoff steht.

Von ganz besonderm Interesse sind weiterhin Verbindungen der Formel (I), in denen
- R¹ und Z: die oben angegebenen Bedeutungen haben und in denen
- Y¹ und Y⁴: für Chlor stehen und
- Y² und Y³: für Wasserstoff stehen.

Von ganz besonderem Interesse sind ebenso Verbindungen, in denen
- Y¹, Y², Y³, Y⁴ und Z: die oben angegebenen Bedeutungen haben und in denen
- R¹: für jeweils an der Verknüpfungsstelle durch Methyl, Ethyl, Chlor oder Brom substituiertes Cyclopentyl, Cyclohexyl, Bicyclo-2,2,1-heptyl oder Bicyclo-2,2,2-octyl, oder
für Cyclopropyl, das an mindestens 2 Positionen durch Chlor und an jeder der anderen drei freien Positionen gegebenenfalls durch Methyl oder Ethyl substituiert ist, oder
für gegebenenfalls einfach bis dreifach durch Fluor oder Chlor substituiertes t-Butyl steht.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Die zur Durchführung des erfindungsgemäßen Verfahrens a) als Ausgangsstoffe benötigten Hydroxyverbindungen sind durch die Formel (II) allgemein definiert. In dieser Formel (II) haben R¹, Y¹, Y², Y³ und Y⁴ vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für R¹, Y¹, Y², Y³ und Y⁴ angegeben wurden.

Die Ausgangsstoffe der Formel (II) sind bekannt und/oder können nach an sich bekannten Verfahren hergestellt werden (vgl. z. B. EP-A 293718, EP-A 339418, EP-A 653418, EP-A 653417, DE-A 19504599).

Die zur Durchführung des erfindungsgemäßen Verfahrens a) weiterhin als Ausgangsstoffe benötigten Halogenverbindungen sind durch die Formel (III) allgemein definiert. In dieser Formel (III) hat Z vorzugsweise bzw. insbesondere diejenigen Bedeutung, die bereits im Zusammenhang mit der Beschreibung der erfindungsgemäßen Verbindungen der Formel (I) als bevorzugt bzw. als insbesondere bevorzugt für Z angegeben wurde. X¹ steht für Halogen, vorzugsweise für Chlor oder Brom.

Die Halogenverbindungen der allgemeinen Formel (III) sind bekannt und/oder können nach an sich bekannten Verfahren hergestellt werden (vgl. z. B. DE-A 2119518, DE-A 2040175, J.Pharm.Sci., 79, 1, 1990, 66-73).

Die zur Durchführung des erfindungsgemäßen Verfahrens b) als Ausgangsstoffe benötigten Aminoverbindungen sind durch die Formel (IV) allgemein definiert. In dieser Formel (IV) haben Y¹, Y², Y³, Y⁴ und Z vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Y¹, Y², Y³, Y⁴ und Z angegeben wurden.

Die Aminoverbindungen der Formel (IV) sind teilweise bekannt und in der Literatur beschrieben worden (vergleiche z. B. J.Chem.Soc., 1961, 1863-1879 und WO-A 96-15118).

Neu und auch Gegenstand der vorliegenden Anmeldung sind Aminoverbindungen der Formel (IV-a) 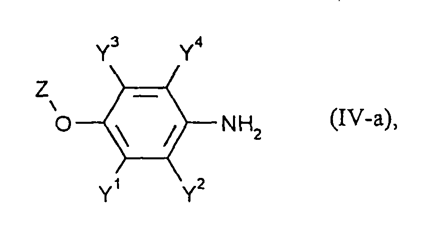 in welcher
- Y¹, Y², Y³, Y⁴ und Z: die oben angegebenen Bedeutungen haben, wobei jedoch mindestens zwei der Reste Y¹, Y², Y³ oder Y⁴ für Halogen stehen.

Die Aminoverbindungen der Formel (IV-a) werden erhalten (Verfahren c), wenn man Aminophenole der allgemeinen Formel (VI) 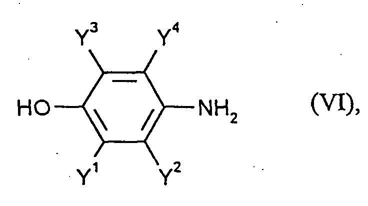 in welcher
- Y¹, Y², Y³ und Y⁴: die oben angegebenen Bedeutungen haben,
mit einer Halogenverbindung der allgemeinen Formel (III)

Z-X¹ (III),

in welcher
- Z: die oben angegebene Bedeutung hat und
- X¹: für Halogen steht,
gegebenenfalls in Gegenwart eines Säureakzeptors und gegebenenfalls in Gegenwart eines Verdünnungsmittels, umsetzt.

Die zur Durchführung des erfindungsgemäßen Verfahrens c) als Ausgangsstoffe benötigten Aminophenole sind durch die Formel (VI) allgemein definiert. In dieser Formel (VI) haben Y¹, Y², Y³ und Y⁴ vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Y¹, Y², Y³ und Y⁴ angegeben wurden.

Die Aminophenole der Formel (VT) sind bekannte Synthesechemikalien.

Die zur Durchführung des erfindungsgemäßen Verfahrens c) weiterhin als Ausgangsstoffe benötigten Halogenverbindungen der Formel (III) sind bereits im Zusammenhang mit der Beschreibung des erfindungsgemäßen Verfahrens a) beschrieben worden.

Als Verdünnungsmittel zur Durchführung der erfindungsgemäßen Verfahren a), b) und c) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid; oder Sulfone, wie Sulfolan.

Die erfindungsgemäßen Verfahren a), b) und c) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydroxide, -acetate, -carbonate oder -hydrogencarbonate, wie beispielsweise Natriumhydroxid, Kaliumhydroxid, Natriumacetat, Kaliumacetat, Calciumacetat, Natriumcarbonat, Kaliumcarbonat, Kaliumhydrogencarbonat oder Natriumhydrogencarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren a), b) und c) in einem größeren Bereich variiert werden. Im allgemeinen arbeitet man bei Temperaturen von -50°C bis 150°C, vorzugsweise bei Temperaturen von -10°C bis 120°C.

Zur Durchführung des erfindungsgemäßen Verfahrens a) zur Herstellung der Verbindungen der Formel (I) setzt man pro Mol der Hydroxyverbindung der Formel (II) im allgemeinen 0,5 bis 5 Mol, vorzugsweise 0,8 bis 2,5 Mol Halogenverbindung der Formel (III) ein.

Zur Durchführung des erfindungsgemäßen Verfahrens b) zur Herstellung der Verbindungen der Formel (I) setzt man pro Mol der Aminoverbindung der Formel (IV) im allgemeinen 0,5 bis 5 Mol, vorzugsweise 0,8 bis 2,5 Mol Carbonsäurederivat der Formel (V) ein.

Zur Durchführung des erfindungsgemäßen Verfahrens c) zur Herstellung der Verbindungen der Formel (IV-a) setzt man pro Mol des Aminophenols der Formel (VI) im allgemeinen 0,5 bis 5 Mol, vorzugsweise 0,8 bis 2,5 Mol Halogenverbindung der Formel (III) ein.

Die Reaktionstemperaturen können bei der Durchführung der erfindungsgemäßen Verfahren a), b) und c) in einem größeren Bereich variiert werden. Im allgemeinen arbeitet man bei Temperaturen von -50°C bis 150°C, vorzugsweise bei Temperaturen von -10°C bis 120°C.

Die erfindungsgemäßen Verfahren a), b) und c) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die Reaktionsdurchführung, Aufarbeitung und Isolierung der Reaktionsprodukte erfolgt nach bekannten Verfahren (vgl. die Herstellungsbeispiele).

Die erfindungsgemäßen Stoffe weisen eine starke mikrobizide Wirkung auf und können zur Bekämpfung von unerwünschten Mikroorganismen, wie Fungi und Bakteria, im Pflanzenschutz und im Materialschutz eingesetzt werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
   (Konidienform: Drechslera, Syn: Helminthosporium);
Cochliobolus-Arten, wie beispielsweise Cochliobolus sativus
   (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei werden die erfindungsgemäßen Wirkstoffe mit besonders gutem Erfolg zur Bekämpfung von Krankheiten im Wein-, Obst- und Gemüseanbau, wie beispielsweise gegen Botrytis-Arten, eingesetzt. Sie weisen ferner eine sehr starke in vitro-Wirkung auf. Ferner lassen sich die erfindungsgemäßen Verbindungen auch zur Steigerung des Ernteertrags von Kulturpflanzen einsetzen.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticdiin-S, Bromuconazol, Bupirimat, Buthiobat,
Calciumpolysulfid, Capsimycin, Captafol, Captan, Carbendazim, Carboxin, Carvon, Chinomethionat(Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilate, Iminoctadinetriacetate, Iodocarb, Ipconazol, Iprobenfos(IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und
B ord eaux-Mi schung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen(PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofosmethyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-β-propyl-1H-1,2,4-triazol-1-ethanol,
α-(2,4-Dichlorphenyl)-β-methoxy-α-methyl-1H-1,2,4-triazol-1-ethanol;
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-α-(Methoxyimino)-N-methyl-2-phenoxy-phenylacetamid,
{2-Methyl-1-[[[1-(4-methylphenyl)ethyl]amino]carbonyl]propyl}-carbaminsäure-1-isopropylester
1-(2,4-Dichlorphenyl)-2-(1H-1,2,4-triazol-1-yl)ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)methoxy]phenyl]ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)phenyl]methyl]-benzamid,
2-(2,3,3-Trüod-2-propenyl)-2H-tetrazol,
2-[(1-Methylethyl)sulfonyl]-5-(trichlormethyl)-1,3,4-thiadiazol,
2-[[6-Deoxy-4-0-(4-0-methyl-β-D-glycopyranosyl)-α-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)oxy]methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(-Methylethyl)-3-methyl-4-[(3-methylbenzoyloxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol, -Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis(2-propinyloxy)-phenyl]-N'-methoxy-methanimidamid,
N-Formyl-N-hydroxy-DL-alanin, -Natriumsalz,
O,O-Diethyl-[2-(dipropylamino)-2-oxoethyl]-ethylphosphoramidothioat,
O-Methyl-S-phenyl-phenylpropylphosphoramidothioat,
S-Methyl-1,2,3-benzothiadiazol-7-carbothioat,
spiro[2H]-1-Benzopyran-2,1'(3'H)-isobenzofuran]-3'-on,

### Bakterizide:

Bromopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb, HCH, Heptenophos, Hexaflumuron, Hexythiazox,
Imidacloprid, Iprobenfos, Isazophos, Isofenphos, Isoprocarb, Isoxathion, Ivermectin,
Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimphos, Teflubenzuron, Tefluthrin, Temephos, Terbam, Terbufos, Tetrachlorvinphos, Thiafenox, Thiodicarb, Thiofanox,
Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihren handelsüblichen Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g, benötigt.
Bei der Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

### Herstellungsbeispiele:

### Beispiel 1

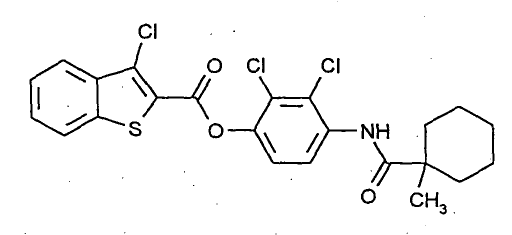

### Verfahren a)

2,1 g (0,007 Mol) 4-(1-Methyl-cyclohexyl)-carbonylamino-2,3-dichlorphenol und 1 ml (0,007 Mol) Triethylamin werden in 30 ml Tetrahydrofuran gelöst. Dann werden bei 0°C 1,61 g (0,007 Mol) 3-Chlorbenzothiophen-2-carbonsäurechlorid, gelöst in 20 ml Tetrahydrofuran, zur Reaktionsmischung getropft. Man erwärmt auf 20°C und gibt dann zur Vervollständigung der Reaktion erneut 0,8 g (3,5 mMol) Carbonsäurechlorid hinzu. Nach Beendigung der Reaktion wird die Reaktionsmischung auf Wasser gegossen, mehrfach mit Essigsäureethylester extrahiert, die organische Phase über Magnesiumsulfat getrocknet und bei vermindertem Druck eingeengt. Der Rückstand wird mit Hexan/Essigsäureethylester/Aceton (30:10:1) an Kieselgel chromatografiert. Man erhält 3,2 g (91% der Theorie) 3-Chlorbenzothiophen-2-carbonsäure-[4-(1-methyl-cyclohexyl)-carbonylamino-2,3-dichlorphenyl]-ester vom Schmelzpunkt 108°C.

### Beispiel 2

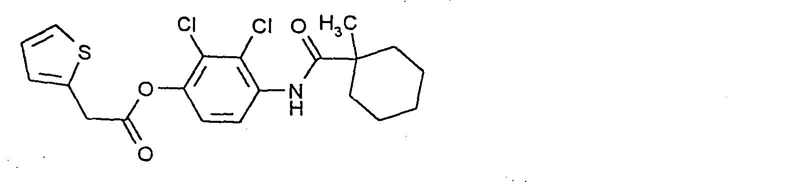

### Verfahren b)

Zu einer Lösung von 0,25 g (0,8273 mMol) Thiophen-2-yl-essigsäure-(4-amino-2,3-dichlorphenyl)-ester in 10 ml Pyridin gibt man nacheinander ca. 50 mg Dimethylaminopyridin und 0,13 g (0,8273 mMol) 1-Methylcyclohexancarbonsäurechlorid und läßt 18 Stunden rühren. Die Reaktionsmischung wird in 200 ml Wasser gegeben, und 3 mal mit jeweils 100 ml Essigsäureethylester extrahiert. Die vereinigten organischen Phasen werden 2 mal mit jeweils 50 ml 1N Salzsäure und einmal mit 50 ml Wasser gewaschen, über Magnesiumsulfat getrocknet und bei vermindertem Druck eingeengt. Der Rückstand wird mit Cyclohexan/Essigester (6:1) an Kieselgel chromatografiert. Man erhält 0,14 g (40 % der Theorie) Thiophen-2-yl-essigsäure-{2,3-dichlor-4-[(1-methyl-cyclohexancarbonyl)-amino]-phenyl}-ester vom Schmelzpunkt 58°C.

### Herstellung des Ausgangsstoffes

### Beispiel (IV-a-1)

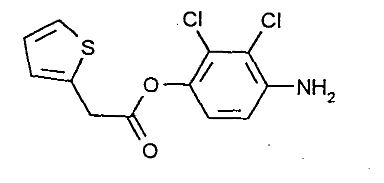

### Verfahren c)

Zu einer Lösung von 1,0 g (5,617 mMol) 2,3-Dichlor-4-Hydroxyanilin in 30 ml Dimethylformamid gibt man unter einer Argonatmosphäre 0,67 g (5,954 mMol) Kalium-t-Butanolat und läßt 5 Stunden rühren. Danach tropft man zu dieser Lösung 0,9 g (5,617 mMol) 2-Thienylacetylchlorid und läßt weitere 15 Stunden rühren. Die Reaktionsmischung wird in 300 ml Wasser gegeben, und 3 mal mit jeweils 100 ml Essigsäureethylester extrahiert. Die vereinigten organischen Phasen werden über Magnesiumsulfat getrocknet und bei vermindertem Druck eingeengt. Der Rückstand wird mit Cyclohexan/Essigester (5:1) an Kieselgel chromatografiert. Man erhält 0,55 g (33% der Theorie) Thiophen-2-yl-essigsäure-(4-amino-2,3-dichlor-phenyl)-ester.
- ¹H-NMR (CDCl₃, TMS):: δ = 6,67 (d, 1H); 6,87 (d, 1H); 7,02 (m, 1H); 7,06 (m, 1H); 7,26 (m, 1H); ppm.

Analog den Herstellungsbeispielen 1 und 2 sowie entsprechend der allgemeinen Beschreibung der erfindungsgemäßen Verfahren a) und b) wurden die in der nachstehenden Tabelle 1 aufgeführten Verbindungen der allgemeinen Formel (Ia) erhalten:

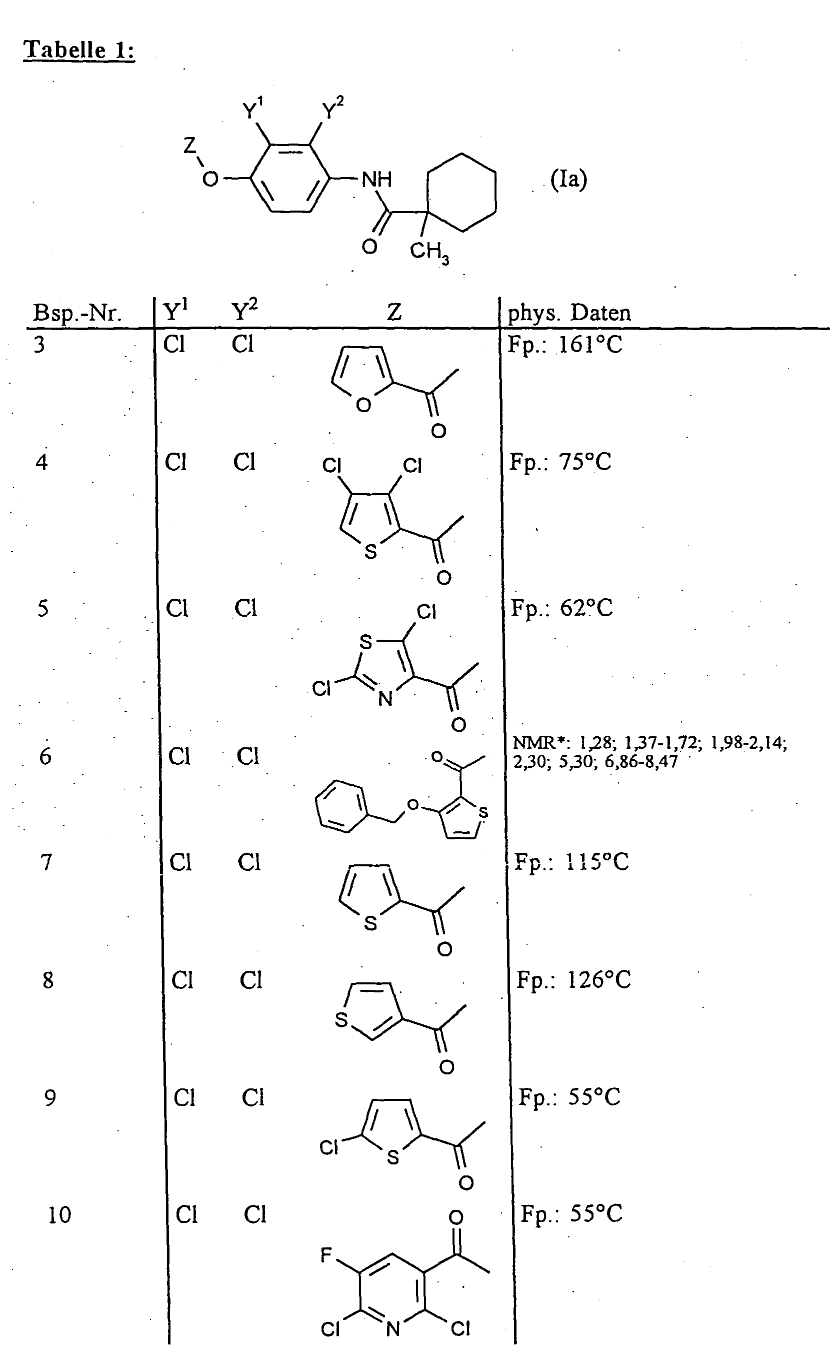

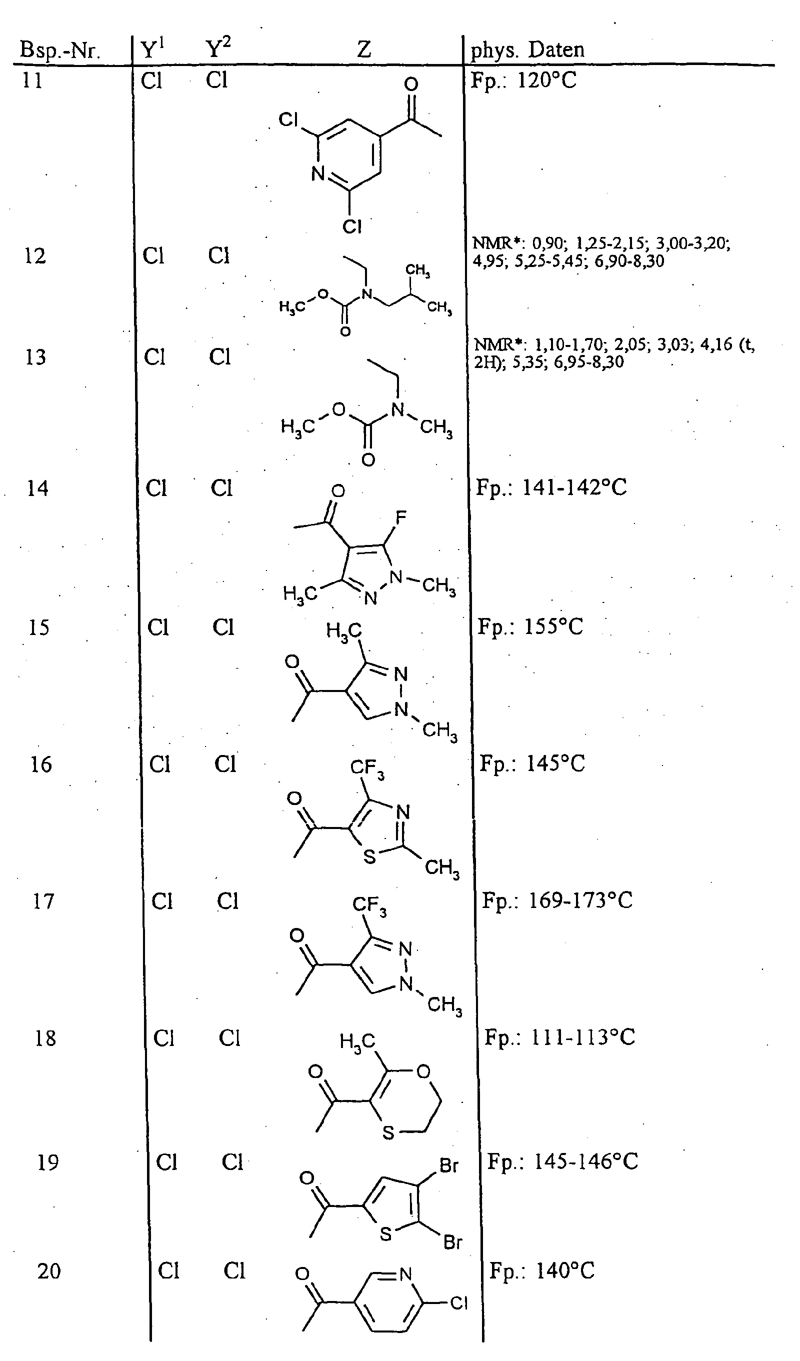

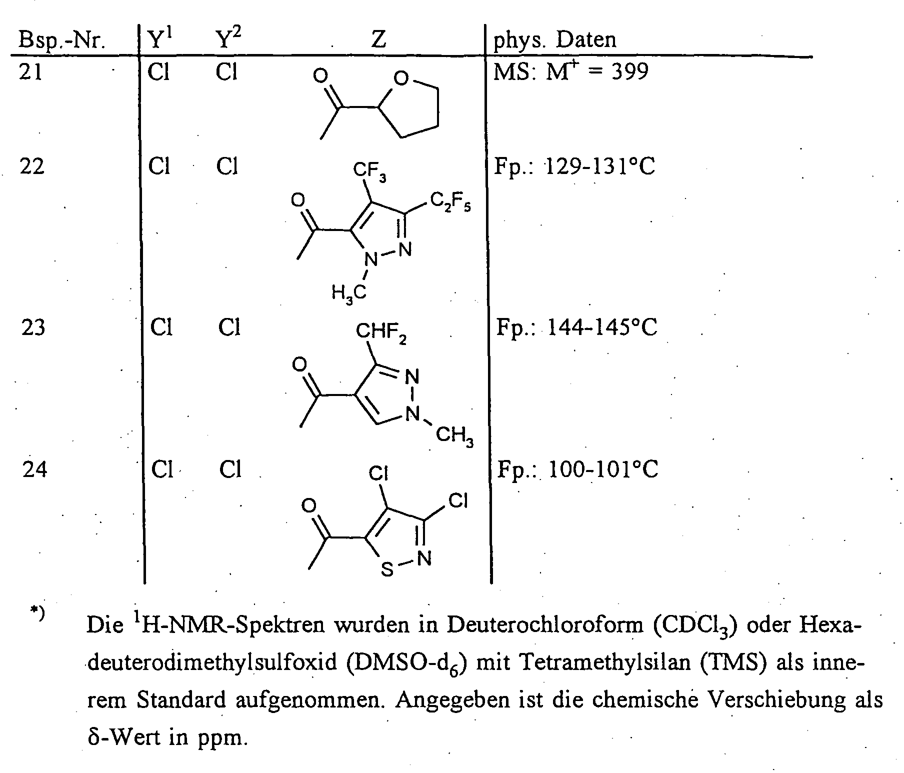

Analog Beispiel (IV-a-1), sowie entsprechend der allgemeinen Beschreibung des erfindungsgemäßen Verfahrens c) wurden die in der nachstehenden Tabelle 2 aufgeführten Verbindungen der allgemeinen Formel (IV-a) erhalten:

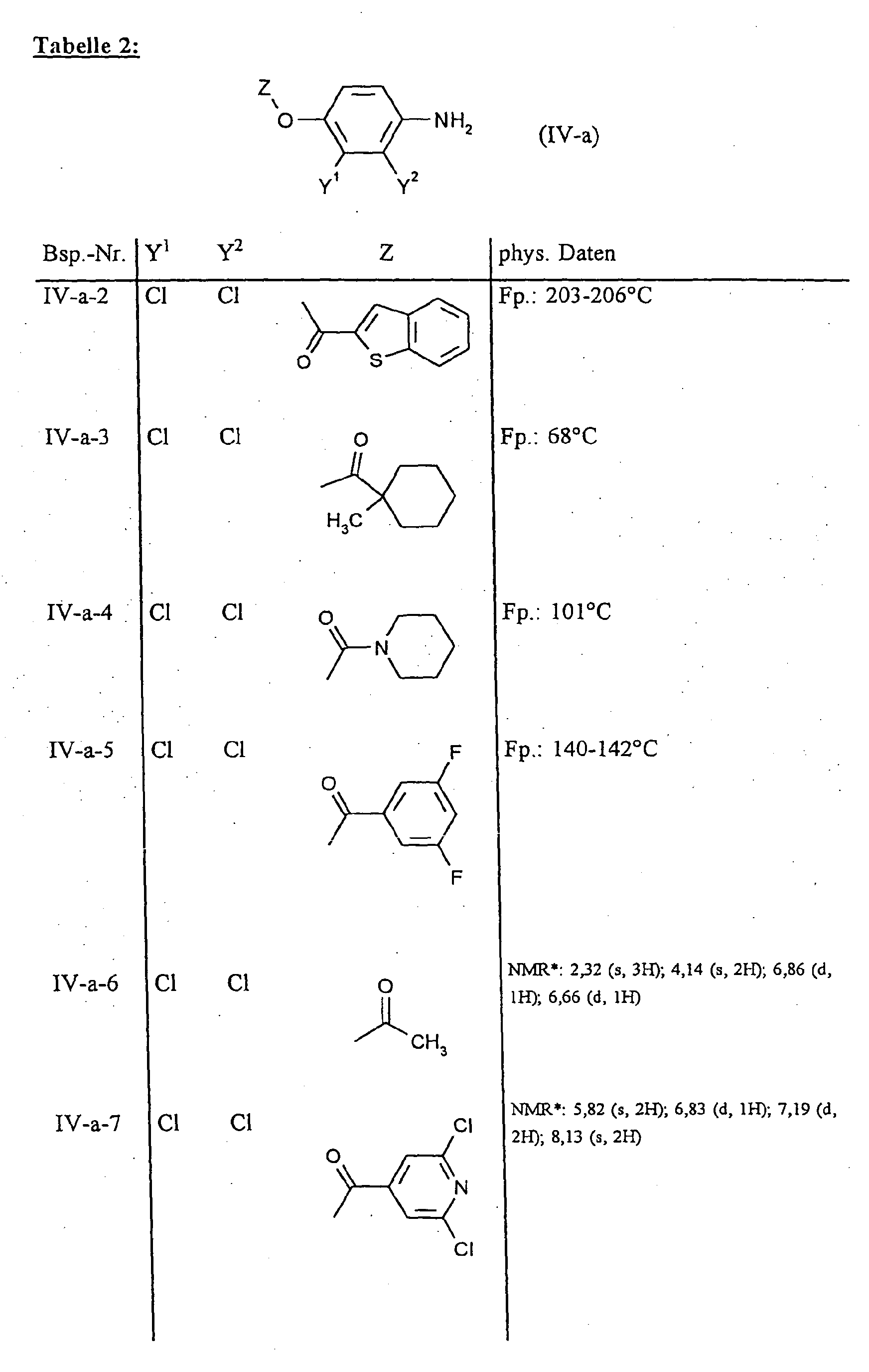

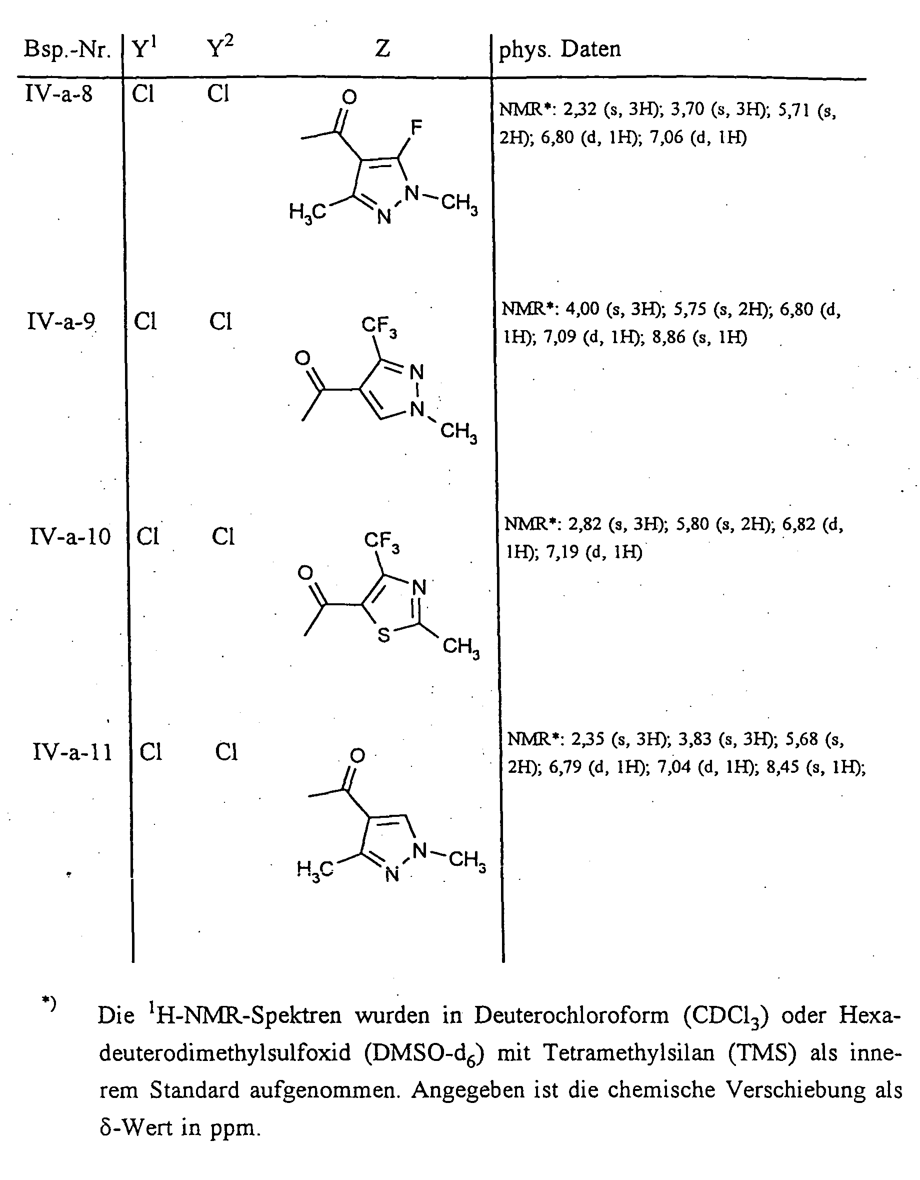

### Anwendungsbeispiele

### Beispiel A

### Botrytis-Test (Bohne) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Bei diesem Test zeigen z.B. die folgenden Verbindungen der Herstellungsbeispiele (2), (3), (4), (5), (7), (8), (9), (12) und (13) bei einer beispielhaften Wirkstoffkonzentration von 100 ppm einen Wirkungsgrad von 69 bis 100%.

## Patentansprüche

1. Verbindungen der Formel (I) 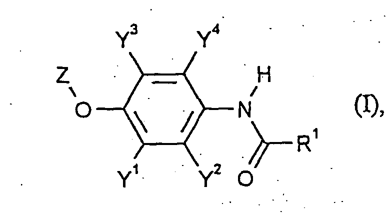 in welcher
Y¹ x für Wasserstoff, Halogen, Alkyl, Alkoxy, Alkylthio mit jeweils 1 bis 4 Kohlenstoffatomen, Halogenalkyl, Halogenalkoxy, Halogenalkylthio mit jeweils 1 bis 3 Kohlenstoffatomen und 1 bis 5 gleichen oder verschiedenen Halogenatomen,
Y² für Wasserstoff, Halogen, Alkyl, Alkoxy, Alkylthio mit jeweils 1 bis 4 Kohlenstoffatomen, Halogenalkyl, Halogenalkoxy, Halogenalkylthio mit jeweils 1 bis 3 Kohlenstoffatomen und 1 bis 5 gleichen oder verschiedenen Halogenatomen,
Y³ für Wasserstoff, Halogen, Alkyl, Alkoxy, Alkylthio mit jeweils 1 bis 4 Kohlenstoffatomen, Halogenalkyl, Halogenalkoxy, Halogenalkylthio mit jeweils 1 bis 3 Kohlenstoffatomen und 1 bis 5 gleichen oder verschiedenen Halogenatomen,
Y⁴ für Wasserstoff, Halogen, Alkyl, Alkoxy, Alkylthio mit jeweils 1 bis 4 Kohlenstoffatomen, Halogenalkyl, Halogenalkoxy, Halogenalkylthio mit jeweils 1 bis 3 Kohlenstoffatomen und 1 bis 5 gleichen oder verschiedenen Halogenatomen,
wobei mindestens zwei der Reste Y¹ bis Y⁴ für Halogen stehen,
R¹ für jeweils gegebenenfalls einfach bis fünffach substituiertes Alkyl oder Alkenyl mit jeweils 4 bis 10 Kohlenstoffatomen steht, und die möglichen Substituenten vorzugsweise aus der nachstehenden Aufzählung ausgewählt sind: Halogen, Alkoxy mit 1 bis 4 Kohlenstoffatomen, Alkylthio mit 1 bis 4 Kohlenstoffatomen, Halogenalkoxy mit 1 bis 2 Kohlenstoffatomen und 1 bis 5 Halogenatomen, Halogenalkylthio mit 1 bis 2 Kohlenstoffatomen und 1 bis 5 Halogenatomen oder gegebenenfalls durch Halogen oder Alkyl substituiertes Cycloalkyl mit 3 bis 6 Kohlenstoffatomen, wobei das Kohlenstoffatom, das an die Carbonylgruppe gebunden ist, kein Wasserstoffatom trägt, oder
R¹ für jeweils gegebenenfalls einfach bis fünffach substituiertes Cycloalkyl oder Cycloalkenyl mit jeweils 3 bis 12 Kohlenstoffatomen steht, und die möglichen Substituenten vorzugsweise aus der nachstehenden Aufzählung ausgewählt sind: Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 2 Kohlenstoffatomen und 1 bis 5 Halogenatomen, Halogenalkoxy mit 1 bis 2 Kohlenstoffatomen und 1 bis 5 Halogenatomen, Halogenalkylthio mit 1 bis 2 Kohlenstoffatomen und 1 bis 5 Halogenatomen oder gegebenenfalls durch Halogen oder Alkyl substituiertes Cycloalkyl mit 3 bis 6 Kohlenstoffatomen, und
Z für eine Gruppierung 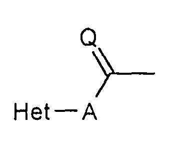 steht,
in welcher
Het für gegebenenfalls einfach bis dreifach durch Halogen, Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen substituiertes Heterocyclyl mit 3 bis 7 Ringgliedern, von denen jeweils 1 bis 3 gleiche oder verschiedene Heteroatome - insbesondere Stickstoff, Sauerstoff und/oder Schwefel - sind, steht,
A für eine Einfachbindung, Sauerstoff, Schwefel, Alkandiyl mit 1 bis 2 Kohlenstoffatomen, oder für 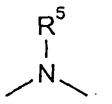 steht,
wobei
R⁵ für Wasserstoff oder Alkyl mit 1 bis 4 Kohlenstoffatomen steht, und
Q für Sauerstoff oder Schwefel steht.

2. Verbindungen der Formel (I) gemäß Anspruch 1,
in welcher
Y¹ für Wasserstoff, Fluor, Chlor, Brom, Methyl, Methoxy, Ethoxy, Methylthio, Ethylthio, Trifluormethyl, Difluormethyl, Fluormethyl, Fluorchlormethyl, Difluormethoxy, Trifluormethoxy, Difluorchlormethoxy, Difluormethylthio, Difluorchlormethylthio, Trifluormethylthio steht,
Y² für Wasserstoff, Fluor, Chlor, Brom, Methyl, Methoxy, Ethoxy, Methylthio, Ethylthio, Trifluormethyl, Difluormethyl, Fluormethyl, Fluorchlormethyl, Difluormethoxy, Trifluormethoxy, Difluorchlormethoxy, Difluormethylthio, Difluorchlormethylthio, Trifluormethylthio steht,
Y³ für Wasserstoff, Fluor, Chlor, Brom, Methyl, Methoxy, Ethoxy, Methylthio, Ethylthio, Trifluormethyl, Difluormethyl, Fluormethyl, Fluorchlormethyl, Difluormethoxy, Trifluormethoxy, Difluorchlormethoxy, Difluormethylthio, Difluorchlormethylthio, Trifluormethylthio steht,
Y⁴ für Wasserstoff, Fluor, Chlor, Brom, Methyl, Methoxy, Ethoxy, Methylthio, Ethylthio, Trifluormethyl, Difluormethyl, Fluormethyl, Fluorchlormethyl, Difluormethoxy, Trifluormethoxy, Difluorchlormethoxy, Difluormethylthio, Difluorchlormethylthio, Trifluormethylthio steht,
wobei mindestens zwei der Reste Y¹ bis Y⁴ jeweils für Fluor, Chlor oder Brom stehen,
R¹ für 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl steht, wobei die genannten Alkylreste gegebenenfalls durch 1 bis 3 Halogenatome, vorzugsweise Fluor, Chlor oder Brom, substituiert sind, oder
R¹ für 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-1-methyl-2-propenyl, 1-Ethyl-2-methyl-1-propenyl und 1-Ethyl-2-methyl-2-propenyl steht, wobei die genannten Alkylreste gegebenenfalls durch 1 bis 3 Halogenatome, vorzugsweise Fluor, Chlor oder Brom, substituiert sind, oder
R¹ für Cyclopropyl, Cyclobutyl, Cyclopentyl, Cyclohexyl, Cycloheptyl, 1-Cyclopentenyl, 2-Cyclopentenyl, 3-Cyclopentenyl, 1,3-Cyclopentadienyl, 1,4-Cyclopentadienyl, 1-Cyclohexenyl, 2-Cyclohexenyl, 3-Cyclohexenyl, 1,3-Cyclohexadienyl, 1,4-Cyclohexadienyl, 1,5-Cyclohexadienyl, 2,4-Cyclohexadienyl, 1-Cycloheptenyl, 2-Cycloheptenyl, 3-Cycloheptenyl, 4-Cycloheptenyl, 1,3-Cycloheptadienyl, 1,4-Cycloheptadienyl, 1,5-Cycloheptadienyl, 1,6-Cycloheptadienyl, 2,4-Cycloheptadienyl, 2,5-Cycloheptadienyl, 2,6-Cycloheptadienyl und 3,5-Cycloheptadienyl steht, wobei die genannten Reste gegebenenfalls durch 1 bis 3 Halogenatome, vorzugsweise Chlor, Fluor oder Brom, und/oder Methyl oder Ethyl substituiert sind, oder
R¹ für Bicyclo-[2.2.1]-hex-5-yl, Bicyclo-[2.2.1]-hept-2-yl, Bicycylo-[2.2.2]-oct-2-yl, Bicyclo-[3.2.1]-oct-6-yl, Bicyclo-[3.2.2]-non-6-yl, Bicyclo-[4.2.2]-dec-7-yl, Bicyclo-[3.1.0]-hex-1-yl, Bicyclo-[4.1.0]-hept-1-yl, Bicyclo-[4.3.0]-non-1-yl, Bicyclo-[4.4.0]-dec-1-yl, besonders bevorzugt für 5-Methyl-bicyclo-[2.1.1]-hex-5-yl, 2-Methyl-bicyclo-[2.2.1]-hept-2-yl, 2-Methyl-bicyclo-[2.2.2]-oct-2-yl, 6-Methyl-bicyclo-[3.2.1]-oct-6-yl, 6-Methyl-bicyclo-[3.2.2]-non-6-yl, 7-Methyl-bicyclo-[4.2.2]-dec-7-yl, 1-Methyl-bicyclo-[3.1.0]-hex-1-yl, 1-Methyl-bicyclo-[4.1.0]-hept-1-yl, 1-Methyl-bicyclo-[4.3.0]-non-1-yl, 1-Methyl-bicyclo-[4.4.0]-dec-1-yl, 2-Methyl-bicyclo-[3.1.0]-hex-1-yl, 2-Methyl-bicyclo-[4.1.0]-hept-1-yl, 2-Methyl-bicyclo-[4.3.0]-non-1-yl, 2-Methyl-bicyclo-[4.4.0]-dec-1-yl, Bicyclo-[2.2.1]-hept-2-en-5-yl, Bicyclo-[2.2.2]-oct-2-en-5-yl, Bicyclo-[4.2.2]-dec-7-en-2-yl, Bicyclo-[4.3.0]-non-7-en-1-yl, Bicyclo-[4.4.0]-dec-3-en-1-yl, Bicyclo-[4.1.0]-hept-3-en-1-yl, 5-Methyl-bicyclo-[2.2.1]-hept-2-en-5-yl, 5-Methyl-bicyclo-[2.2.2]-oct-2-en-5-yl, 2-Methyl-bicyclo-[4.2.2]-dec-7-en-2-yl, 2-Methyl-bicyclo-[4.3.0]-non-7-en-1-yl, 2-Methyl-bicyclo-[4.4.0]-dec-3-en-1-yl und 2-Methyl-bicyclo-[4.1.0]-hept-3-en-1-yl steht,
Z für eine Gruppierung 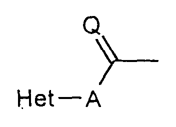 steht,
in welcher
Het für gegebenenfalls einfach bis dreifach durch Halogen, Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen substituiertes Heterocyclyl mit 3 bis 7 Ringgliedern, von denen jeweils 1 bis 3 gleiche oder verschiedene Heteroatome - insbesondere Stickstoff, Sauerstoff und/oder Schwefel - sind, steht,
A für eine Einfachbindung, Sauerstoff, Schwefel, Methylen, oder für 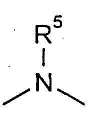 steht,
wobei
R⁵ für Wasserstoff, Methyl oder Ethyl steht, und
Q für Sauerstoff oder Schwefel steht.

3. Schädlingsbekämpfungsmittel, **gekennzeichnet durch** einen Gehalt an mindestens einer Verbindung der Formel (I) nach Anspruch 1.

4. Verfahren zur Bekämpfung von Schädlingen **dadurch gekennzeichnet, daß** man Verbindungen der Formel (I) nach Anspruch 1 auf Schädlinge und/oder ihren Lebensraum einwirken läßt.

5. Verwendung von Verbindungen der Formel (I) nach den Ansprüchen 1 bis 5 zur Bekämpfung von Schädlingen.

6. Verfahren zur Herstellung von Schädlingsbekämpfungsmitteln, **dadurch gekennzeichnet, daß** man Verbindungen der Formel (I) nach den Ansprüchen 1 bis 5 mit Streckmitteln und/oder oberflächenaktiven Mitteln vermischt.

7. Verfahren zur Herstellung von Verbindungen der Formel (I) wie in Anspruch 1 definiert, **dadurch gekennzeichnet, daß** man a) Hydroxyverbindungen der allgemeinen Formel (II) 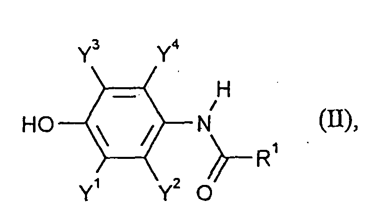 in welcher
R¹, Y¹, Y², Y³ und Y⁴ die in Anspruch 1 angegebenen Bedeutungen haben,
mit einer Halogenverbindung der allgemeinen Formel (III)
Z-X¹ (III),
in welcher
Z die in Anspruch 1 angegebene Bedeutung hat und
X¹ für Halogen steht,
gegebenenfalls in Gegenwart eines Säureakzeptors und gegebenenfalls in Gegenwart eines Verdünnungsmittels, umsetzt, b)
Aminoverbindungen der allgemeinen Formel (IV) 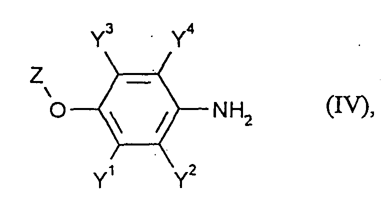 in welcher
Y¹, Y², Y³, Y⁴ und Z die in Anspruch 1 angegebenen Bedeutungen haben,
mit einem Carbonsäurederivat der allgemeinen Formel (V) 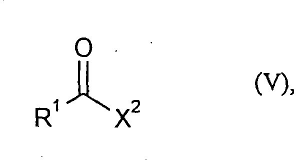 in welcher
R¹ die in Anspruch 1 angegebene Bedeutung hat und
X² für Halogen oder 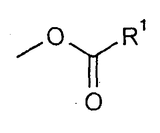 steht,
gegebenenfalls in Gegenwart eines Säureakzeptors und gegebenenfalls in Gegenwart eines Verdünnungsmittels, umsetzt.

8. Verbindungen der Formel (IV-a) 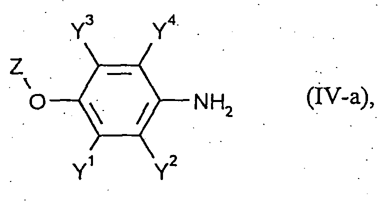 in welcher
Y¹, Y², Y³, Y⁴ und Z die in Anspruch 1 angegebenen Bedeutungen haben, wobei jedoch mindestens zwei der Reste Y¹, Y², Y³ oder Y⁴ für Halogen stehen.
